# EUROPEAN PATENT APPLICATION

(11) **EP 2 700 365 A1**
(43) Date of publication of application: **26.02.2014**
(21) Application number: 12181550.0
(22) Date of filing: 23.08.2012
(51) Int. Cl.: A61B 17/06, A61B 1/04, A61B 19/00

(54) **Suturing toolkit**

(71) Applicant: Nederlandse Organisatie voor Toegepast -Natuurwetenschappelijk Onderzoek TNO, 2628 VK Delft (NL)
(72) Inventor: de Boer, Bart Michiel, 2628 VK Delft (NL); van Neer, Paul Louis Maria Joseph, 2628 VK Delft (NL); Wieringa, Fokko Pieter, 2628 VK Delft (NL); Baan, Jan, 2628 VK Delft (NL)
(74) Representative: Jansen, Cornelis Marinus

(57) **Abstract**

There is provided a suturing toolkit comprising a suture thread comprising suture segments, a camera arranged for recording an image of the suture thread, and an image processor arranged for processing the recorded image and outputting a display image. First and second spectral signatures of different suture segments comprise distinct components in a non-visible wavelength range. The camera is arranged for recording a spectrally resolved image of the distinct components. The image processor is arranged for identifying first image parts in the recorded image that comprise the distinct components of the first spectral signature, identifying second image parts in the recorded image that comprise the distinct components of the second spectral signature, and selectively applying visible contrasting colors to the first and second image parts in the output display image.

## Description

### FIELD AND BACKGROUND OF THE INVENTION

The present invention relates to a suturing toolkit and a method for visualizing a suture.

A suturing toolkit typically comprises a length of suture thread suitable for holding tissues together during and after suturing. The toolkit may further comprise a suture needle that may be integral with the suture thread or be provided separately. Suturing typically comprises steps of knotting the suture thread to secure its position. One problem encountered during suturing, is that a position and relative layout of the suture thread may become difficult to identify.

U.S. Patent No. 6,994,719 B2 discloses a suture strand suitable for use as a suture or ligature comprising a plurality of braided fibers of ultrahigh molecular weight polyethylene, and a plurality of fibers of at least one long chain synthetic polymer or bioabsorbable fiber, the fibers being woven to produce the suture strand, sections of at least one of the plurality of fibers being provided in a color contrasting from the other fibers to provide an identifiable trace along a portion of the suture strand, at least one portion of the suture strand being provided with no identifiable trace.

There is a need for a suturing toolkit and method for visualizing a suture that improves upon known systems in the identification of a relative layout of a suture thread.

### SUMMARY OF THE INVENTION

In a first aspect there is provided a suturing toolkit. The suturing toolkit comprises a suture thread comprising first and second suture segments arranged along a length of the suture thread. A first suture segment comprises a first spectral signature and a second suture segment comprises a second spectral signature. The suturing toolkit further comprises a camera arranged for recording an image of the suture thread. The suturing toolkit further comprises an image processor arranged for processing the recorded image and outputting a display image. Specifically, the first and second spectral signatures comprise distinct components in a non-visible wavelength range. Furthermore, the camera is arranged for recording a spectrally resolved image of the distinct components. Furthermore, the image processor is arranged for identifying first image parts in the recorded image that comprise the distinct components of the first spectral signature, identifying second image parts in the recorded image that comprise the distinct components of the second spectral signature, and selectively applying visible contrasting colors to the first and second image parts in the output display image.

By providing distinct spectral components outside the visible spectrum, these components can be specifically targeted and isolated from visible background components. These non-visible components are normally not observed and may be replaced by visible contrasting colors without interfering with the rest of the visible image. Selected non-visible components may provide additional advantages of increased penetration depth through tissue, thus allowing visualization even when covered by bodily fluids. By applying targeted image processing on a segmented suture thread, different parts of the suture thread may be better distinguished from one another than with either image processing or segmentation of a suture thread separately. Increasing the visible contrast between different segments of a suturing thread provides advantages over increasing a contrast of the suture thread as a whole, e.g. with respect to the background. Particularly, a better identification of the relative layout of the different parts of the suture thread becomes possible. When different suture segments overlap, it may be easier to identify which segment of the suture thread is in front and which is in the back due to the relative contrast between the suture segments. This may aid e.g. knotting the suture thread. An improvement in depth perception may be especially noticeable for a one-dimensional structure such as a suture thread. In addition, when the relative layout of the suture thread can be established, e.g. in relation to known parts of the environment, also a position of the suture thread may be better estimated. Using image processing to image the suture thread allows increasing the visible contrast between different segments of the suture thread beyond a visible contrast that can be achieved by e.g. providing a thread having inherent contrasting colors. Accordingly, there is provided a suturing toolkit that improves upon known systems in the identification of a relative layout of a suture thread.

In a second aspect there is provided a suture thread comprising first and second suture segments arranged along a length of the suture thread. A first suture segment comprises a first spectral signature and a second suture segment comprises a second spectral signature. The first and second spectral signatures comprise distinct components in a non-visible wavelength range. The spectral signatures can e.g. be spectrally resolved from each other by a camera of the suturing toolkit arranged for recording a spectrally resolved image of the suture thread. The suture thread may thus be visualized by said camera to improve identification of a relative layout of the suture thread.

In a third aspect there is provided a method for visualizing a suture thread. The method comprises providing a suture thread comprising first and second suture segments arranged along a length of the suture thread. A first suture segment comprises a first spectral signature and a second suture segment comprises a second spectral signature. The method further comprises providing a camera arranged for recording an image of the suture thread. The method further comprises providing an image processor arranged for processing the recorded image and outputting a display image. Specifically, the first and second spectral signatures comprise distinct components in a non-visible wavelength range. Furthermore, the camera is arranged for recording a spectrally resolved image of the distinct components. Furthermore, the image processor is arranged for identifying first image parts in the recorded image that comprise the distinct components of the first spectral signature, identifying second image parts in the recorded image that comprise the distinct components of the second spectral signature, and selectively applying visible contrasting colors to the first and second image parts in the output display image.

Using this method an improved contrast between different parts of the suture thread may be provided. Accordingly there is provided a method for visualizing a suture that improves upon known systems in the identification of a relative layout of a suture thread.

### BRIEF DESCRIPTION OF THE DRAWINGS

These and other features, aspects, and advantages of the apparatus, systems and methods of the present invention will become better understood from the following description, appended claims, and accompanying drawing wherein:
FIG 1 shows an embodiment of a suturing toolkit;
FIG 2 shows an embodiment of a suturing toolkit comprising a multispectral camera;
FIG 3 shows an embodiment of a suturing toolkit comprising an endoscope;
FIG 4 shows an example of selectively increasing contrast between image parts;
FIG 5 shows an example of dynamically increasing contrast between image parts and a background
FIG 6 shows an example of distinct spectral signatures;
FIG 7 shows an embodiment of a suture thread and needle.

### DETAILED DESCRIPTION

Unless otherwise defined, all terms (including technical and scientific terms) used herein have the same meaning as commonly understood by one of ordinary skill in the art to which this invention belongs as read in the context of the description and drawings. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some instances, detailed descriptions of well-known devices and methods may be omitted so as not to obscure the description of the present systems and methods. Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be further understood that the terms "comprises" and/or "comprising" specify the presence of stated features but do not preclude the presence or addition of one or more other features. All publications, patent applications, patents, and other references mentioned herein are incorporated by reference in their entirety. In case of conflict, the present specification, including definitions, will control.

Color is a visual perceptual property that may depend on a spectral distribution (wavelength and intensity) of light and a perceived interaction of this light with receptors in the (human) eye. Colors may vary in different ways, including hue (e.g. red, green, blue), saturation, and brightness. By defining a color space, colors can be identified numerically by their coordinates. In an absolute color space the perceptual difference or contrast between colors may be related to distances between colors as represented by points in the color space. Examples of absolute color spaces include the sRGB and CIE XYZ color spaces.

Visible contrast also simply referred to as 'contrast', is the perceived distinction between image parts, e.g. perceived by an average human. When visible contrast is increased between image parts, the image parts can be better told apart. Complementary colors, e.g. from a color wheel, may represent high chromatic contrast. In one definition, two colors are called complementary if, when mixed in the proper proportion, they produce a neutral color (grey, white, or black). Examples of complementary colors pairs may include: green-red, purple-yellow, and blue-orange. Also different colors that are not complementary may provide sufficient contrast, e.g. red-yellow. Besides chromatic contrast, there may also be achromatic contrast, depending on the relative intensity, i.e. lightness of the image parts. For example, black-white represent a high level of achromatic contrast. In some cases high contrast may be established by combining complementary coloring and increasing an intensity difference.

Color and contrast may comprise a physiological component as perceived lightness of a color may depend on a sensitivity of the eye to specific colors, e.g. being more sensitive to green light. Color and contrast may also comprise a psychological component. For example, two intense colors may compete for 'attention' in the mind of a user. In such a case, perceived contrast may be enhanced by making one color lighter and the other darker. It is noted that for the present disclosure it is not necessary for achieving a desired effect that the colors are chosen with a maximum contrast, merely for an increased contrast sufficient to unambiguously distinguish between different image parts by an average user.

In one example, a table comprising sets of two of more contrasting colors may be created by recording perceived high contrasts between sample colors, e.g. perceived by a panel of average users. For increasing a visible contrast between image parts, these image parts may be assigned contrasting colors from the table. The assigned colors may depend on a third color, e.g. a background color, to provide not only contrast between the two assigned colors but also with respect to the background. The assigned color may be unrelated to the original color, e.g. with respect to a relative position in color space. An image part that may originally be perceived as having no color (black), may comprise spectral components outside the visible spectrum. Two image parts having different spectral components outside the visible spectrum may be assigned two different visible colors for increasing a visible contrast.

In another example, a multi-dimensional contrast space may be constructed wherein points in the contrast space represent different colors and/or intensities and a distance between the points a degree of contrast between said different colors and/or intensities. Such a contrast space may e.g. comprise an absolute color space, optionally combined with another dimension representing a relative lightness scale. For increasing a visible contrast between image parts, recorded colors and/or intensities of these image parts may be located in the contrast map and new colors and/or intensities may be assigned to the image parts by increasing a distance in the contrast space. In this way the colors that are assigned to the image parts may be related to the original colors, though with increased contrast.

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. The description of the exemplary embodiments is intended to be read in connection with the accompanying drawings, which are to be considered part of the entire written description. In the drawings, the size and relative sizes of systems, components, layers, and regions may be exaggerated for clarity. Embodiments are described with reference to cross-section illustrations that are schematic illustrations of possibly idealized embodiments and intermediate structures of the invention.

In the description, relative terms as well as derivatives thereof should be construed to refer to the orientation as then described or as shown in the drawing under discussion. These relative terms are for convenience of description and do not require that the system be constructed or operated in a particular orientation unless stated otherwise. It will further be understood that when an element or layer is referred to as being "on", "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. It will further be understood that when a particular step of a method is referred to as subsequent to another step, it can directly follow said other step or one or more intermediate steps may be carried out before carrying out the particular step. Like numbers refer to like elements throughout.

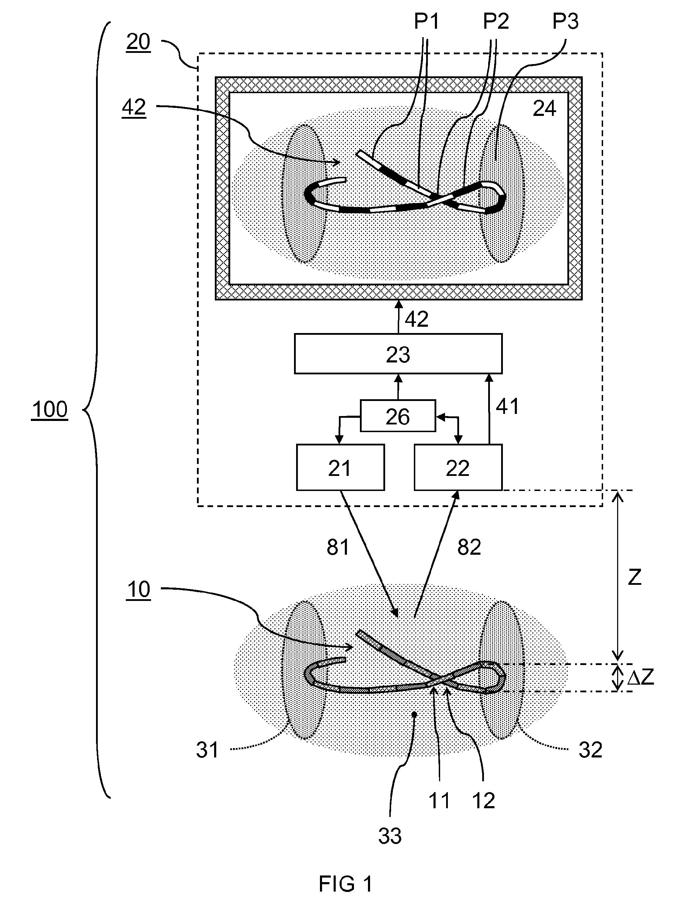

FIG 1 shows a suturing toolkit 100 comprising a suture thread 10 and an imaging system 20 for visualizing the suture thread.

The suture thread 10 comprises a striped pattern of suture segments 11 and 12 arranged along a length of the suture thread 10, also referred to as suture wire. A first suture segment 11 comprises a first spectral signature and a second suture segment 12 comprises a second spectral signature. The second spectral signature is distinct from the first spectral signature, i.e. they can be distinguished by the imaging system. In one example, two spectra may be distinguished when they are linearly independent. This may be the case e.g. when a first spectrum comprises a different distribution of spectral components compared to a second spectrum.

The imaging system 20 comprises a camera 22 arranged for recording a spectrally resolved image 41 of the suture thread 10. In said spectrally resolved image 41, at least the first and second spectral signatures of the suture segments are spectrally resolved from each other to distinguish them. The individual spectral distributions of the spectral signatures can be measured beforehand and stored in a memory of the imaging processor 23. In one example, two spectral signatures may be spectrally resolved if a recorded spectrum can be decomposed to reconstruct the contribution of the individual spectral signatures to the recorded spectrum. This may be more accurate if each spectral signature comprises a strong spectral component in a wavelength region where the other spectral signature has a weak or no spectral component.

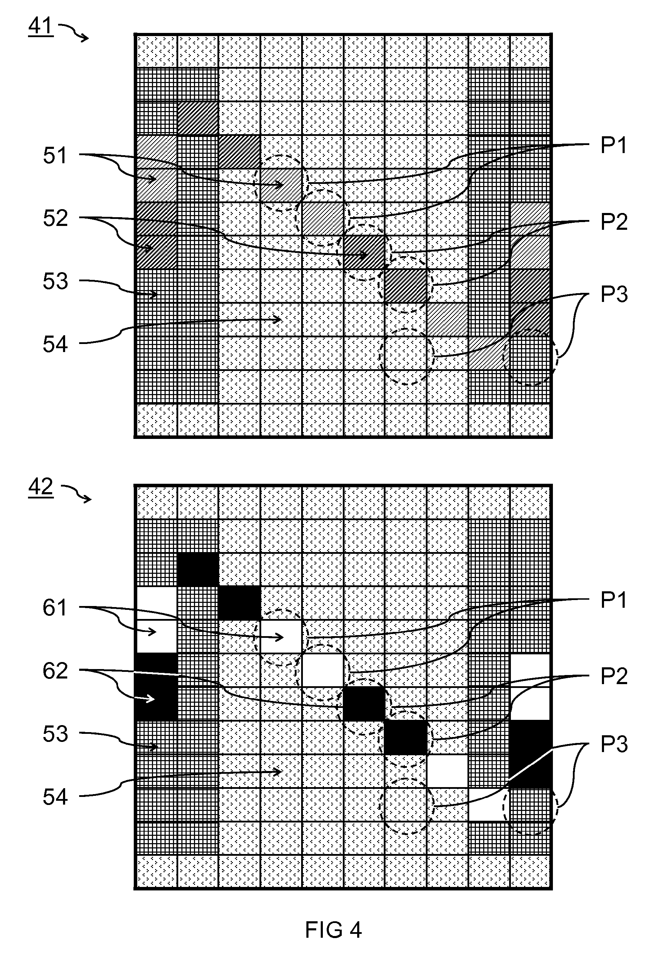

The imaging system 20 further comprises an image processor 23 arranged for processing the recorded image 41 and outputting a display image 42. As will be explained in more detail with reference to FIG 4, the image processor 23 is arranged for identifying first image parts P1 in the recorded image 41 that comprise the first spectral signature and identifying second image parts P2 in the recorded image 41 that comprise the second spectral signature. In said identification, e.g. a threshold value may be used wherein an image part is identified as comprising a spectral signature if the spectral signature is recorded for said image part with an intensity or amplitude above the threshold value. Alternatively or in addition, each image part may be identified as comprising a spectral signature if an amplitude of said spectral signature is higher than amplitudes of other spectral signatures.

The image processor 23 is further arranged for selectively increasing a visible contrast between the first and second image parts P1 and P2 in the output display image 42. The term 'selectively' as used in this context means that the image processor 23 is arranged for specifically increasing the contrast between the first and second image parts. The contrast enhancement may be independent from the rest of the image, e.g. environment image parts P3 corresponding to tissue 32. This may be compared e.g. to general contrast enhancement that typically affects the whole image and does not selectively target a specific subset of image parts with respect to a specific other subset of image parts. E.g. in a general contrast enhancement also a contrast may be enhanced between irrelevant parts of the image. This general contrast enhancement may not satisfy a specific need for being able to distinguish different parts of the suture thread.

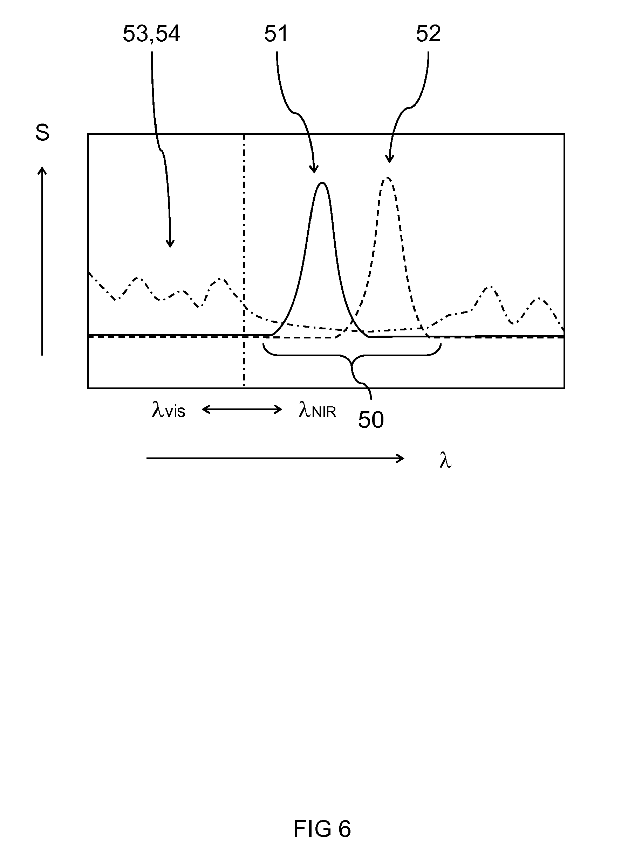

With reference to figures 4 and6, the image processor is arranged for identifying first image parts P1 in the recorded image 41 that comprise the distinct components of the first spectral signature 51. The image processor is further arranged for identifying second image parts P2 in the recorded image 41 that comprise the distinct components of the second spectral signature 52. The image processor is further arranged for selectively applying a first color 61 to the first image parts P1 and a second color 62 to the second image parts P2 wherein the first and second colors 61,62 contrast with each other in the output display image 42.

With continued reference to figure 1, the first and second spectral signatures comprise spectral components that are distinct in a non-visible wavelength range, below 400 nm (UV) or above 700 nm (IR). To record said distinct spectral components, the camera 22 is preferably arranged for recording said components separately in said non-visible wavelength range. Using a non-visible wavelength may have an advantage that it may be distinct from any visible background color. The used wavelength range for the distinct spectral components of the spectral signatures are preferably in a near-infrared (NIR) wavelength range between 0.7 - 1.4 µm. Molar absorptivity in the NIR wavelength range is typically quite small allowing deeper sample penetration than say visible light. Light in an even more preferred wavelength range between 0.7 - 1 µm, may have an advantage of being readily imaged by commonly used and/or cheaper camera sensors.

Emission and/or reflection of light in the NIR by the suture thread 10 may have an advantage that typical tissue materials may be partly or wholly transparent for said light. In this way the suture thread 10 may be recorded through said tissues materials to be displayed on display 24. Also, the suture thread 10 and the suture segments 11, 12 may still be visible on the display 24 even when covered by bodily fluids such as blood, e.g. in an in vivo environment. This may provide advantages over a suture thread only having visible contrasting colors. Accordingly in an advantageous method of using the suturing toolkit 100, the suture thread 10 is irradiated through an obstruction transparent to the illumination light 81, e.g. transmitting more than 10% of the NIR light over a distance of 1 cm though the obstruction. Alternatively to NIR, also other non-visible ranges may be used for achieving similar advantages, e.g. ultraviolet (UV) light between 100 - 400 nm or longer wavelength infrared (IR), e.g. above 2.5 µm. Also combinations of IR, NIR, and/or UV are possible.

In a further embodiment, the camera 22 is arranged for recording the non-visible spectral components of the suture thread 10 and a visible image of the environment 31, 32, 33 of the suture thread. The image processor 23 may be arranged for superimposing the first or second image parts P1, P2 onto the visible image, wherein said first or second image parts P1, P2 are assigned visible contrasting colors. In this way, a suture thread 10 with contrasting segments can be shown on top of a visible image of the background having familiar unchanged colors.

In one embodiment, the image processor 23 may perform a method comprising identifying pixels in a UV/IR image that have registered an intensity in excess of a predefined threshold; identifying pixels in a visible image that correspond to the identified pixels, i.e. registration of the visible and UV/IR image; and changing the color of the registered pixels in the visible image to a high-contrast color and displaying the new visible image as output image. Preferably, though not necessarily, the UV/IR image has the same number of pixels as the visible image.

In an embodiment, the image processor 23 may be arranged for selectively changing colors and/or intensities of recorded first and second image parts P1, P2 into relatively more contrasting visible colors and/or intensities thereby increasing the visible contrast between the first and second image parts as perceived by an average human. In one embodiment, the image processor 23 is arranged for assigning two contrasting colors from a table comprised in a memory of the image processor 23. In another embodiment, the image processor 23 is arranged for shifting a hue and/or lightness of the two image parts in opposite directions, e.g. on an absolute color map comprised in a memory of the image processor 23, for increasing a contrast between the image parts. The contrasting colors assigned to the image parts need not be mono-colored. For example, a lightness or hue of the contrasting colors may be modified gradually as function of amplitude of the recorded spectral signatures to get a more natural shading of the imaged sutured thread.

As an illustration of this principle, the shown suture thread in image 42 (top part of the figure) has a higher contrast between the image parts P1 and P2 compared to a regular image or view of the segments 11 and 12 (bottom part of the figure). It will be appreciated that the higher contrast between image parts makes it easier to estimate a relative layout of the suture thread, in particular which part is in front of the other. It is noted that while in the present figures the contrasting colors are illustrated using black and white, in practice these colors may be any set of contrasting colors.

While the suture thread 10 is shown in this and other figures as having two different types of suture segments, alternatively three or more types of suture segments may be provided each comprising a distinct spectral signature. Additionally, the camera may be arranged for recording more than two distinct spectral signatures of the suture segments and the image processor may be arranged for identifying more than two different image parts and assigning more than two contrasting colors. In one example, a third suture segment comprises a third spectral signature that is distinct from the first and second spectral signatures; the camera is arranged for recording a spectrally resolved image also of the third spectral signature; and the image processor is additionally arranged for identifying third image parts in the recorded image that comprise the distinct components of the third spectral signature; and selectively applying a third color to the third image parts wherein the first, second, and third colors contrast with each other in the output display image.

A distinct spectral signature may e.g. be created for each suture segment by applying a varying combination of multiple dyes. While the suture thread 10 is shown in a preferred embodiment having a plurality of suture segments arranged as a striped alternating pattern along a length of the suture thread 10, alternatively the suture thread may comprise just two parts formed by two suture segments to achieve at least some of the advantages of the present disclosure.

While the suture segments are shown as being approximately equal in length, alternatively, in an embodiment a length of one suture segments is different from a length of another suture segment. The image processor may be arranged for distinguishing between suture segments based on the distance interval. In one example, the first suture segments have one length, e.g. 3 cm while the second suture segments may a different length , e.g. 4 cm. In another example, a length of suture segments at the ending of the suture thread has a different length, e.g. 2 cm, than the suture segments in the middle of the suture thread, e.g. 4 cm. In yet another example, a length of the suture segments may vary across the suture thread according to a chirp, e.g. a length of the suture segments increases or decreases along the thread. Advantage of a variation of length of the suture thread may include application of signal processing techniques to identify a location of the thread e.g. under poor lighting conditions. Also, the length variation may be used for estimating lengths.

In an embodiment the camera is arranged for recording an image of an environment surrounding the suture thread 10. The environment may e.g. comprise background 33 and/or tissue parts 31 and 32 that are to be sutured. The image processor 23 is further arranged for identifying environment image parts P3 in the recorded image 41 that are not identified as first or second image parts P1 and P2. For example, all image parts that do not comprise the first and/or second spectral signatures above a certain threshold value may be identified as environment image parts P3. The image processor 23 is arranged for dynamically increasing a visible contrast between the first image parts P1 with respect to the environment image parts P3. This way at least the first image parts P1 of the suture thread 10 may be better distinguished from the environment. The image processor 23 may further be arranged for dynamically increasing a visible contrast between the second image parts P2 with respect to the environment image parts P3. This way also the second image parts P2 of the suture thread 10 may be better distinguished from the environment.

In one embodiment the image processor 23 is arranged for dynamically increasing a visible contrast between the first image parts P1 with respect to the environment image parts P3 as a function of an average color of the environment image parts P3. Using this image processing, an optimal contrast may be obtained based on the mean or median color of a region of interest around the (suture) pixels, which can be dynamically adapted. The average or median colors may be determined e.g. taking an average or median of RGB values of the colors.

In another embodiment, the contrast is dynamically increased as a function of a color of environment image parts P3 adjacent the first image parts P1. Using this image processing, an optimal contrast may be obtained based on the pixel colors of a region of interest which are weighted according to the distance relative to the pixel of interest. The colors assigned to the segments of the suture thread 10 may optionally vary across a length of the suture thread 10, e.g. if different segments are adjacent to different colored backgrounds. However, preferably the coloring of the suture thread 10 is consistent to avoid confusion.

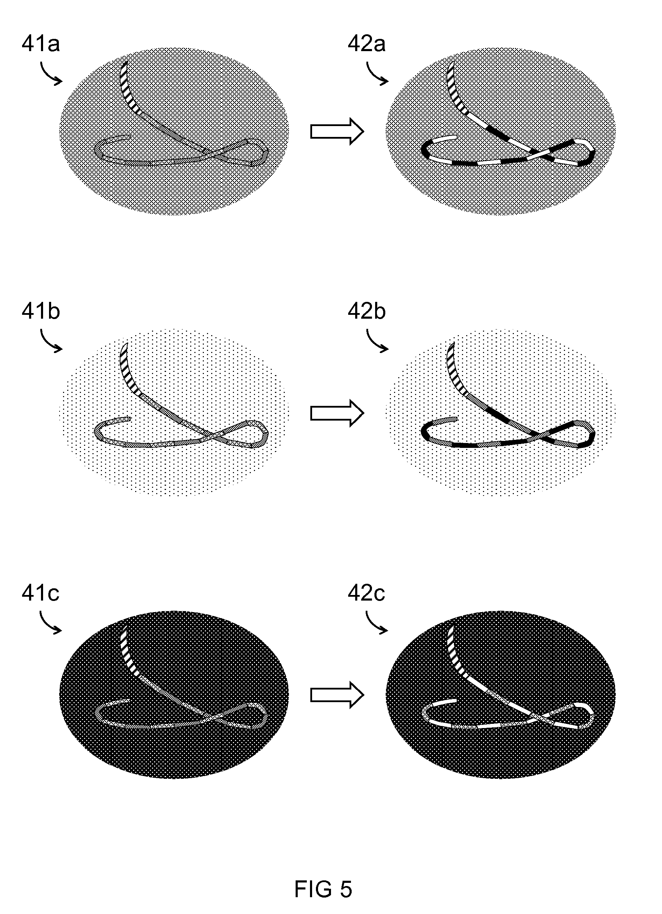

By dynamically increasing a visible contrast, the contrast is adjusted depending on the background 31, and/or tissue 32, 33. The dynamic adjustment may be real-time, e.g. when the camera makes a real-time recording of the suturing process. In each frame of the recording, a background of the suture thread 10 may be identified and the contrast dynamically increased. E.g. when the background is darker, the colors assigned to the suturing thread may be lighter and vice versa. Examples of this are shown in FIG 5. When the background is red, colors assigned to the suturing thread may be yellow and blue, when the background is yellow, colors assigned to the suturing thread may be red and blue, etcetera.

In the shown embodiment, the imaging system 20 further comprises a display 24. The display 24 may receive the output display image 42 from the image processor 23. The output display image 42 may correspond to a signal output of the image processor 23, suitable to be displayed on a display 24, e.g. VGA, DVI, HDMI etcetera. The output display image 42 may also correspond to a signal that can be transformed into an output suitable to be displayed on a display 24, e.g. via intermediate circuitry. A person performing suturing may inspect the display 24 to identify a layout of the suture thread 10, in particular to view the suture segments 11, 12 with sufficient contrast to tell them apart.

In the shown embodiment, the imaging system 20 further comprises a light source 21 arranged for illuminating the suture thread 10 with illumination light 81 thereby directly or indirectly causing emitted light 82 from the illuminated suture thread 10. The emitted light 82 may comprise spectral signatures of the respective suture segments. The spectral signatures may comprise spectral components that result from absorption, transmission, reflection, and/or emission of the respective suture segments. The spectral components may be related to the illumination light 81, e.g. as a selective absorption, transmission and/or reflection of the illumination light 81, and/or the spectral components may be shifted in wavelength with respect to the illumination light 81, e.g. as a result of fluorescent emission. The light source 21 may comprise visible wavelengths and/or non-visible wavelengths. In an embodiment wherein non-visible spectral signatures are used, the light source 21 may comprise dedicated lighting means, e.g. a NIR Light Emitting Device (LED) arranged for providing substantial radiation overlapping said non-visible spectral signatures. Alternatively, a dye in the suture thread 10 may convert visible light to NIR or UV light, e.g. by fluorescence.

It is to be appreciated that having a shifted wavelength may improve a capability of the image processor 23 to distinguish wavelengths emitted from the suture thread 10 from mere reflections, e.g. of the surrounding structures 31 or 32. Accordingly, in an embodiment the suture thread 10 comprises a fluorescent dye wherein the first or second spectral signature comprises a fluorescence spectrum of the fluorescent dye. In a further embodiment, the fluorescent dye has a fluorescence half-life of more than one minute. The suturing thread 10 may e.g. be exposed to illumination light 81 prior to being used for suturing. This way the suture thread 10 may produce its own emitted light 82 during suturing dispensing a need for the light source 21. Accordingly, in an advantageous method the suture comprises a luminescent or fluorescent dye and the illuminating the suture comprises irradiating the suture before using the suture, wherein the dye of the irradiated suture in use emits luminescent or fluorescent radiation. In one example, a fluorescent dye is used that fluoresces for 30 minutes or more.

Some or all of the components of the imaging system 20 may be controlled by a controller 26. The controller 26 and the image processor 23 may be dedicated components or be integrated, e.g. part of a general purpose CPU. Also other components not shown may be part of the imaging system 20. For example, the controller 26 and/or the image processor 23 may have access to an integrated or dedicated memory module comprising e.g. instructions for processing images, a table with contrasting colors, spectral signatures of dyes used for the suture segments, and/or other algorithms and look-up values. Some components may also be split up into multiple components performing different parts of a task.

In an embodiment, the controller 26 may control a timing and/or wavelength output of the light source 21. This timing and/or wavelength output may be coordinated with the camera 22 and/or image processor 23. E.g. light source 21 may be controlled to emit a first wavelength of light during a first time interval and a second wavelength of light during a second time interval. The camera and image processor may record and process spectral responses of the suture thread 10 to different illumination light 81. For example, a first wavelength may be visible and a second wavelength NIR. A single camera that is sensitive to both wavelengths may be used to record a visible image of the background and a NIR image of the suture thread 10. The image processor 23 may combine the images into a single output display image 42. The wavelength of the light may be controlled e.g. by varying parameters of the light source, by applying a filter and/or by changing parameters of a spectrally resolving element such as a grating or prism. The timing of the light may be controlled by a shutter and/or the light source may itself be pulsed, e.g. a laser.

In an embodiment the suturing toolkit 100 as described, comprises means for measuring and/or calculating a three-dimensional layout of the suture thread. The suturing toolkit 100 is thus arranged not only for recording a flat image of the suture thread 10, but also for measuring and/or calculating a third dimension Z of the suture thread 10. Said third dimension Z may be a distance of the suture thread 10 with respect to the camera 22 as indicated. The third dimension Z may represent a depth of the suture thread 10. The depth may vary between suture segments.

In said embodiment, preferably the image processor 23 is further arranged for differentiating between different segments of the suture thread based on the three-dimensional layout and dynamically increasing a visible contrast between different segments of the suture thread as a function of a distance ΔZ between the segments. For example, in a scenario wherein two different suture segments with the same spectral signature are in front of each other, this embodiment may distinguish the segments by a difference ΔZ along a third dimension Z. The contrast may e.g. be increased between overlapping segments by making a suture segment brighter when it is more to the front (smaller Z).

In one embodiment, the means for measuring and/or calculating a three-dimensional layout of the suture thread comprises a time of flight (TOF) recorder arranged for recording an optical time of flight of light 81 emitted by the light source 21, reflected by the suture thread 10 as light 82 and recorded by the camera 22. The TOF recorder may e.g. be comprised in the controller 26 recording a time difference between sending a pulse of light by the light source 21 and a time that said pulse is received by camera 22. A distance Z of the suture thread with respect to the camera can thus be calculated as a function of the recorded TOF. This distance may e.g. be proportional to the recorded time difference.

This embodiment may be combined with a light source 21 arranged for emitting alternating illumination spectra. For example, a first illumination spectrum overlaps the first spectral signature and not the second spectral signature while a second illumination spectrum overlaps the second spectral signature and not the first spectral signature. In this way the separate TOF and distance of the separate suture segments can be recorded. Of course also more than two illumination and corresponding spectral signatures can be used.

In a further embodiment the light source 21 emits light with a wavelength content limited to a part of the spectrum which color codes for a single part of the suture thread. The wavelength content of the light is altered in time to correspond to the peak reflectivity or peak transmittance of different suture segments. Then, an optical time of flight method or stereoscopic triangulation method is applied to measure the distance between different parts of the suture. The distance information at each part of the spectrum is used to color code the suture thread in the image, and thus to dynamically optimize the contrast between different sections of suture thread as well as between the suture thread and the tissue background.

In another embodiment, the means for measuring and/or calculating a three-dimensional layout of the suture thread comprises a light source 21 arranged for projecting a structured lighting pattern illuminating the suture thread under an angle with respect to the camera. The structured lighting pattern may e.g. comprise a series of parallel projected lines with known distance between them. The camera 22 is arranged for recording the structured lighting pattern projected off the illuminated suture thread 10. Due to the angle with respect to the camera, a recorded image of the structured lighting pattern is a function of a three-dimensional layout of the suture thread 10. E.g. in the shown configuration with illumination light 81 projecting downwards to the right, when the suture thread 10 is moved further to the back (larger Z) the structured lighting pattern will shift to the right. The image processor 23 or a dedicated component, e.g. comprised in the controller 26, is arranged for processing the structured lighting pattern for calculating the three-dimensional layout of the suture thread.

In a further embodiment the suture thread 10 is color coded equally along its length by a pigment which has maximum reflective properties in a broad part of the spectrum, e.g. a broad part of the UV/NIR. The light source 21 uses the said structured lighting principle for illumination. The lighting pattern can be implemented passively by a grating or actively by altering the light source. If the lighting pattern is based on a fixed grating, the wavelength of the emitted light is changed to alter the points where the distance can be measured. The illumination wavelength preferably remains within the area of the spectrum wherein the suture pigment has maximum reflective properties. The color coding is used to dynamically optimize the contrast between suture and tissue. The distance information obtained using the structured light is used to dynamically optimize the contrast between different sections of suture.

In yet another embodiment, the means for measuring and/or calculating a three-dimensional layout of the suture thread comprises, the suture segments 11, 12 form a striped pattern on the suture thread 10, wherein the striped pattern has a predetermined distance interval. The image processor 23 comprises 3D reconstruction means arranged for comparing an interval of the striped pattern in the recorded image 41 to the predetermined distance interval. The image processor 23 is further arranged for reconstructing a three-dimensional layout of the suture thread on the basis of said comparing. For example, if is it found that the distance interval of the striped interval is suddenly smaller for part of the suture thread, an algorithm in the image processor 23 may interpret this as the suture thread 10 being at an angle with respect to the camera 22. If the distance interval of the suture segments is consistently larger, this may be interpreted as the suture thread being closer to the camera. If the distance interval of the suture segments is consistently smaller, this may be interpreted as the suture thread being farther away from the camera. These and other embodiments may also be combined, e.g. the TOF embodiment may be combined with measuring a distance interval between stripes. Also other ways of determining a relative depth of the suture thread 10 may be used in combination, e.g. an acoustic echo apparatus may be linked with the image processor 23.

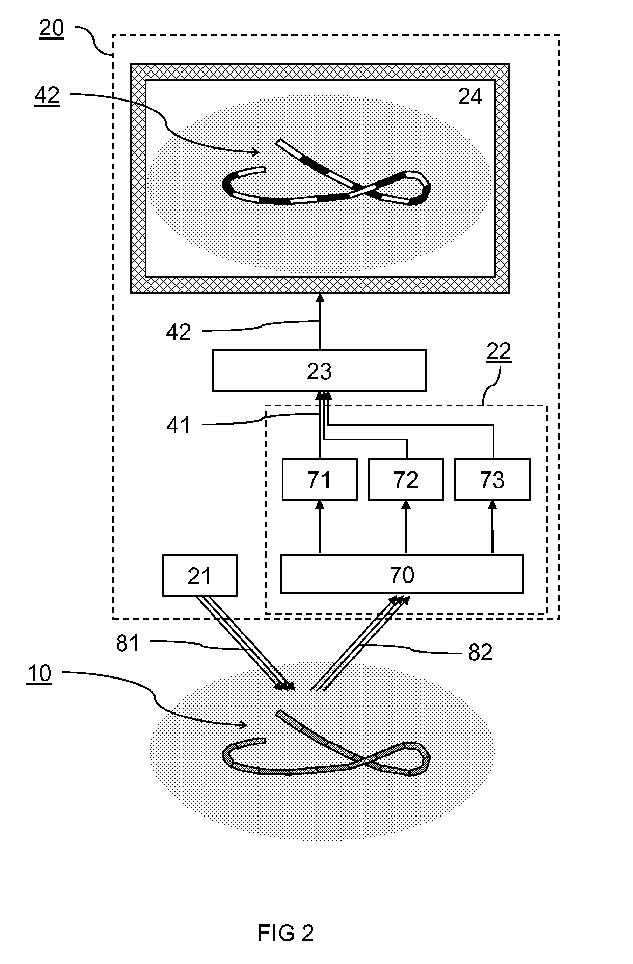

FIG 2 shows an embodiment of a suturing toolkit comprising a camera 22 arranged for processing multispectral light. The camera 22 comprises a spectrally resolving element 70 arranged for spectrally resolving multispectral light 82 received by the camera 22. The camera may comprise multiple sensor elements 71, 72, 73 for recording the spectrally resolved light. E.g. the multispectral light 82 may be split into different wavelength ranges by the spectrally resolving element 70. The spectrally resolving element 70 may e.g. comprise two or more filters which pass different wavelength ranges of multispectral light 82 onto sensors 71, 72, 73. Alternatively or in addition, the spectrally resolving element 70 may comprise diffracting optics such as a diffraction grating and/or dispersing optics such as a prism.

In use, the light source 21 is arranged for emitting multispectral light 81 illuminating the suture thread 10. The emitted light 82 may comprise multispectral components, e.g. reflected spectral components of the illumination light 81 and/or light that is shifted in wavelength with respect to illumination light 81, e.g. as a result of fluorescence of a dye comprised by the suture thread 10. Multispectral light 82, emitted by the suture thread 10, is received by spectrally resolving element 70 and split into wavelength ranges to be received by sensors 71, 72, and 73. Signals from the sensors may be sent to image processor 23 to be combined into output display image 42.

In one embodiment, separate sensors are used for recording an image of non-visible spectral components of the suture thread 10 and visible spectral components of a background of the suture thread 10. E.g. sensor 71 may be a visible light sensor such as found in a color camera. The sensor 71 may be split into further colors, e.g. red, green, and blue. Sensor 72 may be arranged for measuring an image of light with a first non-visible spectral signature and sensor 73 may be arranged for measuring an image of light with a second non-visible spectral signature.

The sensors 71, 72, and 73 may comprise any combination of sensors or sensing elements capable of measuring the respective spectral components of the suture thread and/or environment impinging the sensor or its sensing elements, e.g. pixels. The sensors may comprise any suitable photo sensor or detector for detecting the relevant impinging electromagnetic radiation. Examples may include active pixel sensors (e.g. CMOS), charge-coupled devices (CCD), photo resistors or light dependent resistors (LDR), photovoltaic cells, photodiodes, photomultiplier tubes, phototransistors, or combinations thereof. To filter specific wavelengths, each sensor may comprise an integrated filter instead of or in addition to the spectrally resolving element 70.

Alternative to using dedicated sensors, a time-varying spectrally resolving element 70 and/or filters may be used to vary wavelengths sent to the sensor. The different wavelengths may be combined by the image processor 23. In addition to a sensor also lenses or other focusing means such as parabolic mirrors may be comprises in the camera 22 to create an image of the suture thread 10 and/or the environment onto respective sensors.

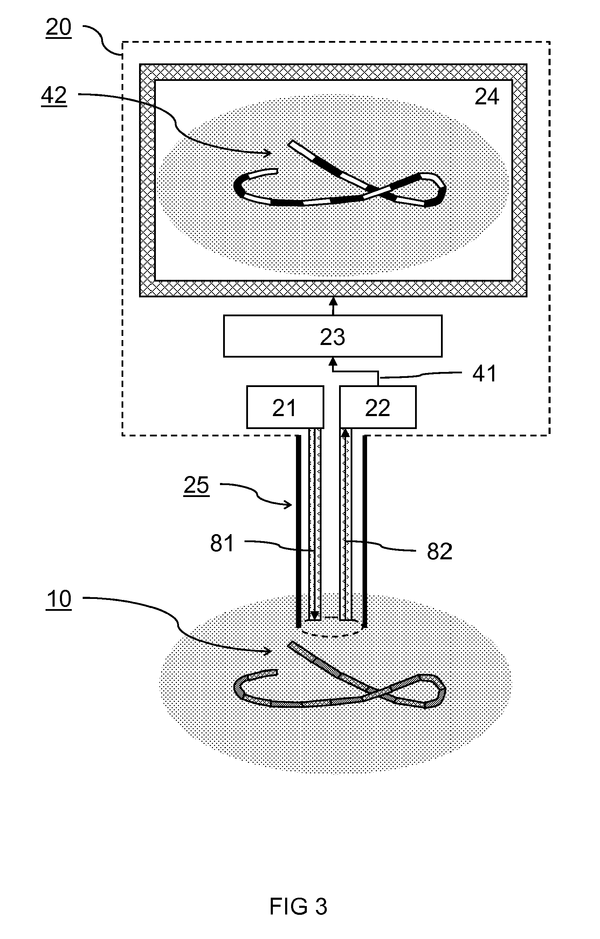

FIG 3 shows an embodiment of a suturing toolkit 100, wherein the imaging system 20 comprises an endoscopic tube 25. The camera 22 is arranged for recording the image 41 of the suture thread 10 via the endoscopic tube 25. In the shown embodiment, the endoscopic tube 25 comprises light guiding means 81 for guiding light from the light source 21 to the suture thread 10 and light guiding means 82 for guiding light from the suture thread 10 back to the camera 22. The light guiding means may e.g. comprise optical fibers or a series of lenses.

It is recognized that an increasing percentage of medical procedures is performed in a minimally or non-invasive manner, e.g. through small openings in the human body (laparoscopy). Endoscopic cameras may be employed to allow observing a region of interest inside a body. It will be appreciated that the present disclosure may provide advantages for a camera operating via an endoscopic tube. In particular, an endoscope typically provides a monoscopic view making it hard to estimate a depth and/or relative layout of a suture thread, especially when the suture thread is of a single color, fluorescent or otherwise. And even if a multicolored suture thread is used, this thread may become covered in bodily fluids such as blood.

One aspect of the present disclosure may be to enhance the contrast between two suture segments, and/or the contrast between the suture thread and surrounding tissue, such that the suturing can be performed with reduced effort and in a shorter time. By being able to tell different suture segments apart, it can be established which part of the suture thread is in front of the other, which may provide a sense of depth. It will be appreciated that the present disclosure may offer advantages for invasive and non-invasive suturing, i.e. stitching of human or non-human body tissues, living or non-living, for cosmetic, therapeutic or other purposes. Advantages of using the presently disclosed toolkit or imaging method may include reduced scarring and shortened recovery time which may reduce medical costs. Further advantages may include easier, more convenient suturing and shorter average suturing time which may reduce medical cost.

FIG 4 shows an example of selectively increasing contrast between image parts. Different spectral signatures 51, 52, 53, and 54 are illustrated with different fill patterns. Spectral signatures 51 and 52 correspond to first and second suture segments. Spectral signatures 53 and 54 correspond to different background tissue structures. Such as described above the image processor is arranged for identifying first image parts P1 in the recorded image 41 that comprise the distinct components of the first spectral signature 51. The image processor is further arranged for identifying second image parts P2 in the recorded image 41 that comprise the distinct components of the second spectral signature 52. The image processor is further arranged for selectively applying a first color 61 to the first image parts P1 and a second color 62 to the second image parts P2 wherein the first and second colors 61,62 contrast with each other in the output display image 42.

In the shown embodiment the first image parts P1 are formed by a plurality of first pixels comprising the first spectral signature 51 and the second image parts P2 are formed by a plurality of second pixels comprising the second spectral signature 52. The image processor 23 may be arranged for assigning contrasting colors to the first and second pixels P1 and P2, in this case white and black respectively. Image parts P3 not comprising either of the spectral signatures 51 or 52 may be left unaffected in the output display image 42. Alternatively, also these image parts P3 may be altered, e.g. by making them darker or lighter for further increasing a contrast between the suture thread and the background. In one embodiment, the background image may be displayed in black and white while the first and second image parts are displayed with contrasting colors such as red and green. Alternatively or in addition to pixels, the image parts may be formed by other data structures, e.g. vectors drawings.

With reference to FIG 4 in combination with FIG 1, there is provided a method for visualizing a suture thread 10. The method comprises providing the suture thread 10 comprising suture segments 11, 12 arranged along a length of the suture thread 10. A first suture segment 11 comprises a first spectral signature 51 and a second suture segment 12 comprises a second spectral signature 52 distinct from the first spectral signature 51. The method further comprises providing a camera 22 arranged for recording a spectrally resolved image 41 of the suture thread 10 wherein the first and second spectral signatures 51, 52 are spectrally resolved from each other. The method further comprises providing an image processor 23 arranged for processing the recorded image 41 and outputting a display image 42. Specifically the image processor 23 is arranged for identifying first image parts P1 in the recorded image 41 that comprise the first spectral signature 51. The image processor 23 is further arranged for identifying second image parts P2 in the recorded image 41 that comprise the second spectral signature 52. The image processor 23 is further arranged for increasing a visible contrast between the first and second image parts P1, P2 in the output display image 42.

FIG 5 shows examples of dynamically increasing contrast between image parts and a background. Dynamic contrast enhancement enhances not only the contrast between the suture segments but also the contrast of the suture segments with respect to the background. While the principle is presently illustrated with shades of gray colors, in practice, more vivid real colors may be used.

In a first example, recorded image 41a comprises an average gray background. In the corresponding display image 42a, the suture segments are assigned contrasting black and white colors that are clearly visible with respect to the background. In a second example, recorded image 41b comprises a light background. In the corresponding display image 42b, the suture segments are assigned contrasting dark gray and black colors that are clearly visible with respect to the background. In a third example, recorded image 41c comprises a dark background. In the corresponding display image 42c, the suture segments are assigned contrasting light gray and white colors that are clearly visible with respect to the background.

FIG 6 shows an example of distinct spectral signatures 51 and 52. An amplitude S of the spectral signatures is shown as a function of wavelength λ. In particular, the first and second spectral signatures 51 and 52 comprise distinct spectral components in a non-visible wavelength range 50. In the present example, the non-visible wavelength range 50 is comprised in a near infrared wavelength range λNIR above a visible wavelength range λvis. A spectral band of the first spectral signature 51 is at a different wavelength than a spectral band of the second spectral signature 52. The amplitude differences may allow a straightforward distinction between the spectral signatures, e.g. with a bandpass filter centered on either band. Also illustrated in this figure is a typical low contribution of background spectral signatures 53 and 54 in the NIR wavelength range. This low contribution may simplify distinction of the spectral signatures 51 and 52. Optionally, a low absorption of light by background tissue may allow imaging through said tissue.

The amplitude S may e.g. comprise an absorption, transmission, reflection or emission amplitude (or combinations thereof) of the suture segments and/or background. In one example, a suture thread may be distinguished from the background by having a higher reflection or emission in certain wavelength ranges than the background. The suture thread is thus imaged as bright spots on the detector in said wavelength ranges. Alternatively, the suture thread may be distinguished from the background if it has a higher absorption than the background in certain wavelength ranges. In this case the suture segment can be imaged as dark spots on the detector in said wavelength ranges.

The spectral signatures 51 and 52 may e.g. comprise emission and/or reflection spectra of the first and second suture segments of the suture thread, respectively. The spectrum may be caused by the material of the suture thread and/or or by dyes added to the suture thread. Preferably, any dyes used are non-toxic to make them suitable for suturing biological tissue. In one embodiment the suture thread is coded with one or more luminescent or fluorescent dye(s), preferably emitting in the near-infrared. This may allow visualizing the path of the suture thread underneath a tissue surface and/or when the suture thread is covered with bodily fluids. Examples of suitable fluorescent dyes may include Indocyanin Green and Methylene Blue.

Other suitable dyes for the suture segments may include autofluorescent substances, which naturally occur in cells, applied in high concentration to the suture thread. As the concentration of these substances is low in cells the contrast between suture and cells may be high. Since these substances naturally occur in the body, they may be less or non-toxic. Examples may include nicotinamide adenine dinucleotide (NADH), with an excitation maximum at 365 nanometer (nm) and an emission maximum at 450 nm, and Flavin adenine dinucleotide (FAD) with excitation maxima at 360 nm and 450 nm and a single emission maximum at 520 nm. Other examples of suitable dyes may include dyes conventionally used for labeling of biological samples, e.g. biocompatible organic fluorescent dyes such as the cyanine family of dyes, e.g. indocyanine green, squaraine dyes, thiazine and oxazine dyes. Other examples of dyes may include Quantum dots (QD). Most QDs are nanoscale semiconductors with tunable emission wavelengths. By increasing the size of a QD, the emission wavelength is increased as well. Similarly, (single-walled) carbon nanotubes may be used for creating a well defined emission wavelength.

In one embodiment, a suture segment comprises a dual-emissive imaging agent with distinct emission wavelengths both in a visible and non-visible wavelength range. This may allow for combined visible assessment and sensitive camera based imaging. An example may be multispectral InP/ZnS quantum dots (QDs) that exhibit a bright visible green/yellow exciton emission combined with a long-lived far red defect emission. The intensity of the latter emission can be enhanced by pre-irradiation with gamma rays or X-rays and may allow for: inverted QD density dependent defect emission intensity; showing improved efficacies at lower QD densities; and detection without direct illumination and interference from auto-fluorescence.

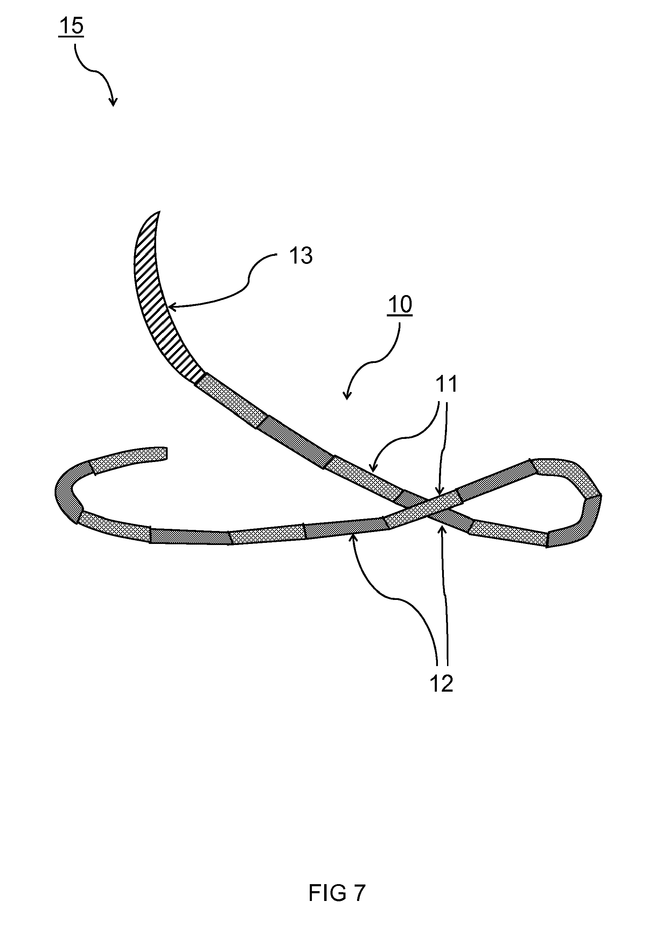

FIG 7 shows an embodiment of a suture 15 comprising a suture thread 10 and suture needle 13. The suture thread 10 is arranged and adapted for use in a suturing toolkit 100 according to any of the previous claims. The suture thread 10 comprises suture segments 11,12 arranged along a length of the suture thread 10. A first suture segment 11 comprises a first spectral signature and a second suture segment 12 comprises a second spectral signature distinct from the first spectral signature.

A suture thread preferably comprises biologically suitable, i.e. non-toxic materials e.g. dyes. Furthermore, the suture thread preferably has sufficient tensile strength for holding tissues together, at least when first applied. In one example, an untied suture thread preferably allows a force of at least ten Newton over a period of one day without breaking. Optionally, the suture thread may dissolve over time. Also, the suture thread preferably has suitable knot tie down characteristics, e.g. being flexible and providing sufficient friction to hold the knot.

In an embodiment, also the suture needle 13 is color coded to dynamically optimize image contrast, especially beneath tissue surface. In the NIR range optical penetration in tissue is typically higher than in the visual range, because most tissue components absorb much less in the NIR. In the NIR range sharp contrasts caused by strong absorption in tissue may be rare. Coating a metal surface with a (patterned) strong absorber, e.g. carbon, may provide a readily identifiable NIR contrast pattern that may also be visualized below the tissue surface.

Accordingly, in an embodiment the suturing toolkit may comprise a suture needle 13 comprising a coating with an absorbing material, wherein the coating is arranged for absorbing more than 25% of incoming near infrared radiation, e.g. in a range of 0.7 **-** 300 µm, preferably at least in a range of 0.7 - 1 µm. In addition to the suture needle 13, also other instruments comprised in a suturing toolkit may be color coded. Said instruments may include scissors (e.g. for holding the needle), scalpels, clamps, staples, etcetera.

In an embodiment the suture thread 10 is color coded using multiple (N) pigments, each pigment having maximum reflective and absorptive properties in a different part of the NIR spectrum, the UV spectrum or a combination of the NIR and UV spectra. In another embodiment a fluorescent suture is color coded using multiple (N) pigments, each pigment having maximum emission in a different part of the NIR spectrum, the UV spectrum or a combination of the NIR and UV spectra. There may thus be a plurality of distinct spectral signatures allowing a detailed identification and mapping of the segments of the suture thread. In an embodiment the imaging system (camera and image processor) is arranged for recording and identifying each of a plurality of segments of the suture thread on a basis of a plurality of distinct spectral signatures of each of the plurality of segments; and increasing a contrast between segments that are in proximity to each other in an output image.

In an embodiment, the suture thread comprises a surface upon which retro reflectors are micro imprinted. In a further embodiment, the suture thread comprises a surface upon which a pattern of wavelength selective retro reflectors is micro imprinted. Micro imprinting may allow creating specific spectral signatures for color coding the suture thread.

While example suturing toolkits and components were shown, also alternative systems and means may be used for achieving similar results. E.g. optical components may be combined or split up into one or more alternative components having similar results. Similarly, electrical components may be split into separate and/or dedicated components or be comprised in integrated circuitry. The various elements of the embodiments as discussed and shown offer certain advantages, such providing a contrasting suture thread. Of course, it is to be appreciated that any one of the above embodiments or processes may be combined with one or more other embodiments or processes to provide even further improvements in finding and matching designs and advantages. It is appreciated that this invention offers particular advantages to suturing tissue, and in general can be applied for any application wherein a layout of a thread needs to be identified.

Finally, the above-discussion is intended to be merely illustrative of the present system and should not be construed as limiting the appended claims to any particular embodiment or group of embodiments. Thus, while the present system has been described in particular detail with reference to specific exemplary embodiments thereof, it should also be appreciated that numerous modifications and alternative embodiments may be devised by those having ordinary skill in the art without departing from the scope of the present systems and methods as set forth in the claims that follow. The specification and drawings are accordingly to be regarded in an illustrative manner and are not intended to limit the scope of the appended claims.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. The mere fact that certain measures are recited in mutually different claims does not indicate that a combination of these measures cannot be used to advantage.

## Claims

1. Suturing toolkit (100) comprising
- a suture thread (10) comprising first and second suture segments (11,12) arranged along a length of the suture thread (10), wherein a first suture segment (11) comprises a first spectral signature (51) and a second suture segment (12) comprises a second spectral signature (52);
- a camera (22) arranged for recording an image (41) of the suture thread (10); and
- an image processor (23) arranged for processing the recorded image (41) and outputting a display image (42); wherein
- the first and second spectral signatures (51,52) comprise distinct components in a non-visible wavelength range (50);
- the camera (22) is arranged for recording a spectrally resolved image (41) of the distinct components; and
- the image processor (23) is arranged for
○ identifying first image parts (P1) in the recorded image (41) that comprise the distinct components of the first spectral signature (51);
○ identifying second image parts (P2) in the recorded image (41) that comprise the distinct components of the second spectral signature (52); and
○ selectively applying a first color (61) to the first image parts (P1) and a second color (62) to the second image parts (P2) wherein the first and second colors (61,62) contrast with each other in the output display image (42).

2. Suturing toolkit (100) according to claim 1, wherein said non-visible wavelength range (50)) is comprised in a near infrared (NIR) wavelength range.

3. Suturing toolkit (100) according to claim 1, wherein said non-visible wavelength range (50)) is comprised in an ultraviolet (UV) wavelength range.

4. Suturing toolkit (100) according to any of the previous claims, wherein
- the camera (22) is further arranged for recording a visible image of an environment (31,32,33) of the suture thread (10); and
- the image processor (23) is arranged for superimposing the first or second image parts (P1,P2) onto the visible image in the output display image (42).

5. Suturing toolkit (100) according to any of the previous claims, wherein
- the camera is further arranged for recording an image of an environment (31,32,33) of the suture thread (10); and
- the image processor (23) is further arranged for
○ identifying environment image parts (P3) in the recorded image (41) that are not identified as first or second image parts (P1,P2); and
○ dynamically increasing a visible contrast between the first image parts (P1) with respect to the environment image parts (P3).

6. Suturing toolkit (100) according to any of the previous claims, wherein the suture thread (10) comprises a fluorescent dye wherein the first or second spectral signature (51,52) comprises a fluorescence spectrum of the fluorescent dye.

7. Suturing toolkit (100) according to any of the previous claims, further comprising a suture needle comprising a coating with an absorbing material, wherein the coating is arranged for absorbing more than 25% of incoming near infrared radiation.

8. Suturing toolkit (100) according to any of the previous claims, wherein the imaging system (20) comprises an endoscopic tube (25), wherein
the camera (22) is arranged for recording the image (41) via the endoscopic tube (25).

9. Suturing toolkit (100) according to any of the previous claims, further comprising means for measuring and/or calculating a three-dimensional layout of the suture thread; wherein the image processor (23) is further arranged for
○ differentiating between different segments of the suture thread based on the three-dimensional layout; and
○ dynamically increasing a visible contrast between different segments of the suture thread as a function of a distance between the segments.

10. Suturing toolkit (100) according to claim 9, wherein the means for measuring and/or calculating a three-dimensional layout of the suture thread comprises
- a time of flight recorder arranged for recording a time of flight (TOF) of light emitted by a light source (21), reflected by the suture thread (10) and recorded by the camera (22); wherein a distance of the suture thread with respect to the camera is calculated as a function of the recorded time of flight (TOF).

11. Suturing toolkit (100) according to claim 10, wherein
- the light source (21) is arranged for emitting alternating illumination spectra, wherein
○ a first illumination spectrum overlaps the first spectral signature and not the second spectral signature; and
○ a second illumination spectrum overlaps the second spectral signature and not the first spectral signature.

12. Suturing toolkit (100) according to claim 9, wherein the means for measuring and/or calculating a three-dimensional layout of the suture thread comprises
- a light source arranged for projecting a structured lighting pattern illuminating the suture thread under an angle with respect to the camera; and
- the camera is arranged for recording the structured lighting pattern projected off the illuminated suture thread; wherein
- the image processor (23) is arranged for processing the structured lighting pattern for calculating the three-dimensional layout of the suture thread.

13. Suturing toolkit (100) according to claim 9, wherein
- the suture segments (11,12) form a striped pattern on the suture thread (10), wherein the striped pattern has a predetermined distance interval; and
- the image processor (23) comprises 3D reconstruction means arranged for
○ comparing an interval of the striped pattern in the recorded image (41) to the predetermined distance interval;
○ reconstructing a three-dimensional layout of the suture thread on the basis of said comparing.

14. Suturing toolkit (100) according to claim 13 wherein the predetermined distance interval is different for different suture segments; and wherein the image processor is arranged for distinguishing one suture segment from another suture segment based on a difference in the predetermined distance interval.

15. Suture thread (10) comprising first and second suture segments (11,12) arranged along a length of the suture thread (10), wherein a first suture segment (11) comprises a first spectral signature (51) and a second suture segment (12) comprises a second spectral signature (52); wherein the first and second spectral signatures (51,52) comprise distinct components in a non-visible wavelength range (50).

16. Method for visualizing a suture thread (10) comprising
- providing a suture thread (10) comprising first and second suture segments (11,12) arranged along a length of the suture thread (10), wherein a first suture segment (11) comprises a first spectral signature (51) and a second suture segment (12) comprises a second spectral signature (52);
- providing a camera (22) arranged for recording an image (41) of the suture thread (10); and
- providing an image processor (23) arranged for processing the recorded image (41) and outputting a display image (42); wherein
- the first and second spectral signatures (51,52) comprise distinct components in a non-visible wavelength range (50);
- the camera (22) is arranged for recording a spectrally resolved image (41) of the distinct components; and
- the image processor (23) is arranged for
○ identifying first image parts (P1) in the recorded image (41) that comprise the distinct components of the first spectral signature (51);
○ identifying second image parts (P2) in the recorded image (41) that comprise the distinct components of the second spectral signature (52); and
○ selectively applying a first color (61) to the first image parts (P1) and a second color (62) to the second image parts (P2) wherein the first and second colors (61,62) contrast with each other in the output display image (42).
